(19) Europäisches Patentamt
European Patent Office
Office européen des brevets

(11) **EP 3 470 839 A1**

(12) **EUROPEAN PATENT APPLICATION**

(43) Date of publication:
**17.04.2019 Bulletin 2019/16**

(51) Int Cl.:
***G01N 33/49*** *(2006.01)*    ***G01N 33/487*** *(2006.01)*
***G01N 33/72*** *(2006.01)*

(21) Application number: **17196065.1**

(22) Date of filing: **12.10.2017**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **Koninklijke Philips N.V.**
**5656 AE Eindhoven (NL)**

(72) Inventors:
• **ARPUTHA, Shashidharan**
**5656 AE Eindhoven (NL)**

• **NARAYANAN, Ratheesh**
**5656 AE Eindhoven (NL)**
• **BHAT, Ravindra**
**5656 AE Eindhoven (NL)**
• **RADHAKRISHNAN, Ravindranath**
**5656 AE Eindhoven (NL)**
• **PUTHANVEEDU VASUDEVAN, Mohandas**
**5656 AE Eindhoven (NL)**

(74) Representative: **de Haan, Poul Erik et al**
**Philips International B.V.**
**Philips Intellectual Property & Standards**
**High Tech Campus 5**
**5656 AE Eindhoven (NL)**

(54) **A SYSTEM AND A METHOD FOR MEASURING BILIRUBIN AND HAEMOGLOBIN**

(57) The present invention relates to a system for measuring bilirubin and haemoglobin of a blood sample. The system of the invention essentially comprises a strip and a reader. The strip of the system is provided for separating plasma and Red Blood Cells (RBCs) in the blood sample. The strip is foldable to form a first portion and a second portion and capable of positioning the first portion over the second portion thereof. The first portion of the strip has a first membrane and the second portion of the strip has a second membrane, attached thereto. The reader of the system is provided for measuring bilirubin or haemoglobin level or both.

The invention also relates to a strip for separating plasma and RBCs in a blood sample, and to a method for measuring bilirubin and haemoglobin of a blood sample.

Fig. 1

EP 3 470 839 A1

**Description**

FIELD OF THE INVENTION

The invention relates to the measurement of bilirubin and haemoglobin. More particularly to a system and a method for measuring bilirubin and haemoglobin, purporting to a point of care device.

BACKGROUND OF THE INVENTION

The breakdown of Red Blood Cells (RBCs) in the body occurs as a natural phenomenon, and is usually compensated by the body by producing more RBCs. Generally the breakdown of haemoglobin in the RBCs is associated with the production of bilirubin in the body. In the event that the rate of breakdown of RBCs exceeds the rate at which the RBCs are produced by the body, a condition called anaemia can develop. The breakdown of haemoglobin is characterized by the production of bilirubin as a breakdown product of haemoglobin. In other words, bilirubin is produced in and by the body when the older RBCs are replaced with the newer RBCs. The liver primarily helps break down the bilirubin, which is usually removed from the body in the stool.

However, under certain conditions, the bilirubin can accumulate and lead to jaundice. Usually Jaundice is characterized by a yellowish or greenish pigmentation of the skin and whites of the eyes due to high bilirubin levels. Jaundice occurs in the first week following birth in over half of neonates. In most cases is not a problem. If the bilirubin levels in neonates are very high for too long, a type of brain damage, known as kernicterus, may occur. The causes of jaundice may vary from being non-serious to potentially fatal. Therefore, it is recommended to check the neonate for jaundice, after the birth and while still in the hospital. The American Academy of Pediatrics (AAP) recommends checking the neonate for jaundice after the birth and again in 3 to 5 days of age, since the bilirubin level is considered to be at its high during this time.

The check for jaundice in the neonate is performed with one or more tests viz. blood test, physical examination and skin test. During physical examination, the body of the neonate is checked for any signs or symptoms of jaundice. Here, the main symptom of jaundice includes yellowish discoloration of the white area of the eye and of the skin. Sometimes urine may be dark in color. On the other hand, the skin test is performed using a dedicated device over the skin that measures the reflection of light with a specific wavelength, which attributes to jaundice. Yet, the blood test is considered to be the best and most reliable amongst all tests to measure bilirubin levels.

It may be appreciated that there exists a correlation between anaemia and bilirubin level, by virtue of the breakdown of haemoglobin and of the RBCs. Hence, it becomes imperative to measure the bilirubin and haemoglobin to ascertain the medical condition holistically.

Known art teaches a device and a method for determining the plasma bilirubin level especially in neonates. It discloses a partially overlapping membrane and filter, where the plasma filtered through the filter reaches the membrane through lateral movement of the plasma effected by capillarity, and gets absorbed therein in the membrane. Further, it teaches that the strip dimensions become critical considering the time required for saturating the membrane with the plasma, which is directly affected by the haematocrit and filter dimensions. Whereas haematocrit being the percentage volume of RBCs, influences the time taken for the plasma to get filtered through the filter provided for the purpose. Also, the strip disclosed therein may have different surface area that require saturation times that are unsuitable for the type of measurement, even leading to false measurements in relation to the haematocrit concentration.

The measurement of bilirubin or haemoglobin or both may be required to be performed depending on the need, and based on the correlation established therein with respect to these two measurements.

There are solutions that are available to measure either of the bilirubin or haemoglobin level. However, the significance of the surface area affecting the saturation time and of the measurement in itself is not unimportant. Besides this, solidification of the RBCs or of the blood on the surface of the filter largely impacts or affects the measurements as such.

Hence there is a need for a solution that overcomes the limitations described above, and can measure the bilirubin and haemoglobin levels more reliably and with simplicity.

The invention is aimed at providing a solution that enables for measuring bilirubin or haemoglobin or both. Moreover the solidification of the RBCs on the surface of the membrane affects the measurement of haemogobin level progressively rather than hindering the measurement.

SUMMARY OF THE INVENTION

Accordingly, the present invention provides a system for measuring bilirubin and haemoglobin of a blood sample. The system of the invention essentially comprises a strip and a reader. The strip of the system is provided for separating plasma and Red Blood Cells (RBCs) in the blood sample. The strip is foldable to form a first portion and a second portion

and capable of positioning the first portion over the second portion thereof. The first portion and second portion of the strip, each has a membrane attached thereto. The reader of the system is provided for measuring bilirubin or haemoglobin level or both.

According to one embodiment of the invention, the reader of the system of the invention has a plurality of light sources. The light sources are provided for illuminating the first portion or the second portion of the strip, for measuring the haemoglobin or bilirubin respectively. The measurement of haemoglobin or bilirubin is based on reflectance photo spectrometry.

According to other embodiment of the invention, the light source provides illumination with blue and green light for measuring bilirubin.

According to another embodiment of the invention, the light source provides illumination with green and red light for measuring haemoglobin.

According to further embodiment of the invention, the reader of the system of the invention has a processing unit. The processing unit is configured to measure the reflectance of the light reflected from the surface of the first portion or second portion of the strip containing RBCs or plasma respectively.

According to yet another embodiment of the invention, the processing unit is provided for estimating the bilirubin and / or haemoglobin.

Accordingly, the invention also provides a strip for separating plasma and RBCs in a blood sample. The strip of the invention comprises a base substrate, a first portion and a second portion. The base substrate of the strip is foldable to form a first portion and a second portion, and capable of positioning the first portion over the second portion thereof. The first portion and second portion, each has a membrane attached thereto to the base substrate by a suitable adhesive

According to one aspect of the invention, the first portion of the strip has a membrane for filtering the plasma in the blood and for allowing the plasma to flow through it. The membrane of the first portion of the strip is also provided for retaining the RBCs in the blood on its surface, and allowing only the plasma to pass through it.

According to another aspect of the invention, the second portion of the strip has a membrane to absorb and retain the plasma.

According to yet another aspect of the invention, the membrane of the strip is a glass microfiber membrane.

Accordingly, the invention also provides a method for measuring bilirubin and haemoglobin of a blood sample. The method of the invention essentially comprises the steps of separating plasma and Red Blood Cells (RBCs) in the blood sample, and measuring bilirubin or haemoglobin level or both. The step of separating plasma and Red Blood Cells (RBCs) in the blood sample is performed by the strip of the system of the invention. The step of measuring bilirubin or haemoglobin level or both is performed by the reader of the invention.

According to one embodiment of the invention, the step of separating plasma and RBCs include filtering the plasma in the blood and allowing the plasma to flow through the membrane of the first portion of the strip. Also, the step of separating plasma and RBCs include retaining the RBCs in the blood on the surface of the membrane of the first portion of the strip and allowing only the plasma to pass through it.

According to other embodiment of the invention, the step of separating plasma and RBCs include absorbing and retaining the plasma by the membrane of the second portion of the strip.

According to another embodiment of the invention, the step of measuring bilirubin or haemoglobin includes illuminating the first portion or the second portion of the strip by a plurality of light sources. It includes illuminating the first portion of the strip with green and red light for measuring haemoglobin. Also, it includes illuminating the second portion of the strip with blue and green light for measuring haemoglobin.

According to yet another embodiment of the invention, the step of measuring the bilirubin and haemoglobin includes measuring the reflectance of the light reflected from the surface of the first portion or second portion of the strip containing RBCs or plasma respectively, by the processing unit of the reader.

According to further embodiment of the invention, the step of measuring the bilirubin and haemoglobin includes estimating the bilirubin and / or haemoglobin by the processing unit.

BRIEF DESCRIPTION OF THE DRAWINGS

With reference to the accompanying drawings in which:

Fig. 1 illustrates a system for measuring bilirubin and haemoglobin, in accordance with the invention;
Fig. 2 shows an exploded view of a strip for separating plasma and RBCs, in accordance with the invention;
Fig. 3 shows the strip of Fig. 2 in a folded position;
Fig. 4 shows a reader for measuring bilirubin and haemoglobin, in accordance with the invention;
Fig. 5 depicts the illumination of the strip for bilirubin measurement;
Fig. 6 depicts the illumination of the strip for anaemia measurement;

Fig. 7 represents a method for measuring bilirubin and haemoglobin, in accordance with the invention;
Fig. 8 depicts an error histogram for bilirubin measurement; and
Fig. 9 depicts an error histogram for haemoglobin measurement.

## DETAILED DESCRIPTION OF EMBODIMENTS

The invention proposes a strip that is foldable and having two portions viz. first and second portion. The first portion may be positioned over the second portion for separating the plasma and the RBCs of the blood sample. The first and the second portion of the strip may be separated for further use with the reader to measure the bilirubin and the haemoglobin levels. The first portion of the strip is used for the measurement of haemoglobin, while the second portion of the strip is used for the measurement of bilirubin. The actual measurement of the bilirubin and the haemoglobin is performed by the reader using on reflectance photo spectrometry. A light source for illuminating the membrane area of the first or the second portion of the strip is provided. The reflectance of the reflected light from the surface of these membranes is used in the estimation of the bilirubin or haemoglobin level, as the case may be.

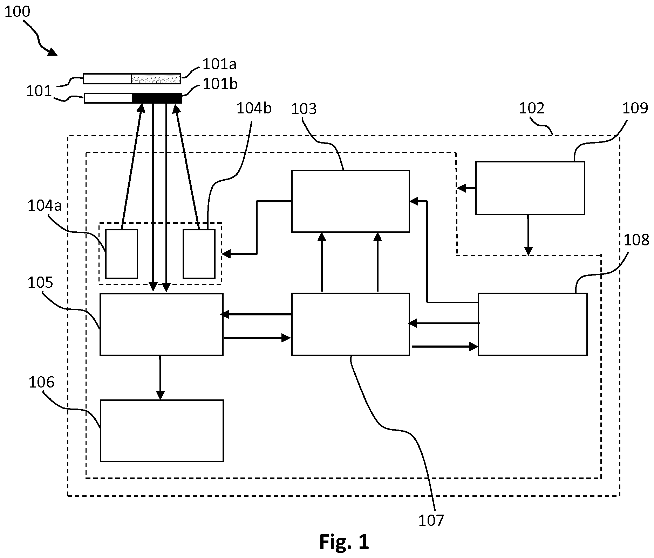

The invention is further described in accordance with a non-exhaustive exemplary embodiment with reference to Figs. 1 to 7. The reference numerals appearing across the figures and throughout the description refer and correspond to the aspects described in the context of the invention.

In Fig. 1, a system (100) for measuring bilirubin and haemoglobin of a blood sample is shown. The system (100) has a strip (101) and a reader (102). The strip (101) is provided for separating the plasma and the RBCs of the blood sample administered to it. The strip is foldable along the its lateral axis, and is composed of a first portion (101 a) and a second portion (101b) that respectively caters to the measurement of haemoglobin and bilirubin. The first portion (101a) and second portion (101b) can be separated from each other for ease of use in the measurement by the reader (102).

The reader (102) has a light source power control (103) for providing power to the light sources (104a, 104b). The light sources (104a, 104b) may be a light source that is suitable for emitting light on the strip, especially on the region of interest. In one embodiment, the light sources (104a, 104b) are Light Emitting Diodes (LEDs), however, other types of light sources are possible. Such light emanating from the light source (104a, 104b) is required to be suitable for the purpose of measurement of the bilirubin or haemoglobin, as the case may be, based on the reflectance photo spectrometry.

The light source 104a is adapted to emit red or blue color. Whereas the light source 104b emits green light. For the measurement of bilirubin, the blue and green lights are used from the light sources (104a, 104b). Whereas for the measurement of haemoglobin, red and green lights provided by the light sources (104a, 104b) are used. A separate light source for emitting red or blue light may be employed instead of a single light source (104a).

The corresponding lights, respective to the measurement of the bilirubin or haemoglobin, illuminates the region of interest on the strip having the RBCs or the plasma. The light emanating from these light sources (104a, 104b) is reflected back from the surface of the region of interest. The intensity of the reflected light is measured by the light to frequency converter (105). The measurements are further processed by the processing unit (106). The processing unit (106) is provided to estimate the bilirubin and / or haemoglobin level.

The reader (102) further has a wireless communication module (107) such as Bluetooth etc. to establish communication between the reader (102) and other devices such as a handheld device like smart phone etc., in its proximity or vicinity. The communication may also be established using wired means. Transfer of data or information pertaining to the measurements or estimated bilirubin or haemoglobin levels or any other information related thereto may be communicated for further use, processing or display. The reader (102) further has a display unit and a user interface (108) which may be made available as a single component or otherwise. The user interface such as but not limited to touch pad or the like may be provided to establish interface with the user to input data or for such other purpose. A power management component (109) is provided to supply and manage power for the operation of the reader (102), and may be provided with a rechargeable battery or the like. The power management component (109) may provide an uninterrupted power supply for a smooth and continuous operation of the reader (102). The power management component (109) may include but limited to a rechargeable battery or the like.

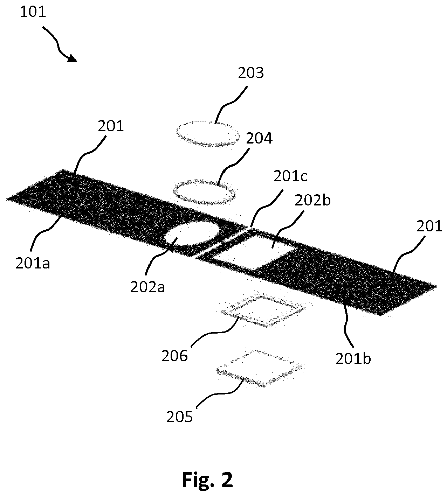

Fig. 2 shows a strip (101) for separating plasma and RBCs in a blood sample administered to it. The blood sample required here may be as little as approximately 30 $\mu$l to measure both haemoglobin as well as bilirubin.

The strip (101) has a base substrate (201) that may be of a material such as a poly film. The base substrate (201) is primarily formed of two portions namely a first portion (201a) and a second portion (201b). The base substrate (201) of the strip (101) is foldable along the lateral axis at a location (201c). The first portion (201a) has a cut out portion (202a) to suitably accommodate a first membrane (203). The first membrane (203) is attached to the first portion (201a) of the base substrate (201) by a suitable adhesive (204). The first membrane (203) having a preferred diameter of 10 mm may be made of glass microfiber, which serves as a filter to separate the plasma and the RBCs of the blood sample administered on top of it or to its surface. The preferred pore size of the glass microfiber may be 2 to 4 $\mu$m. The first

membrane (203) allows only the plasma to flow through it and retain the RBCs on its surface. The RBCs retained on the surface of the first membrane (203) may solidify to a solid state.

Similarly, the second portion (201b) has a selected portion (202b) in the base substrate (201) to suitably accommodate a second membrane (205) that is attached thereto to the second portion (201b) by a suitable adhesive (206). The adhesives (204 and 206) referred herein may be selected from a same group or different group of material and composition depending on the need, purpose and suitability. The second membrane (205) may be made of glass microfiber. The second membrane (205) retains the plasma that been filtered and passed through the membrane (203).

The first portion (201a) of the strip (101), particularly the region of interest purporting to be essentially the surface of the first membrane (203) retaining the RBCs, is used for the measurement of haemoglobin by the reader based on the reflectance photo spectrometry.

Similarly, the second portion (201b) of the strip (101), particularly the region of interest purporting to be essentially the second membrane (205) retaining the plasma, is used for the measurement of bilirubin by the reader based on the reflectance photo spectrometry.

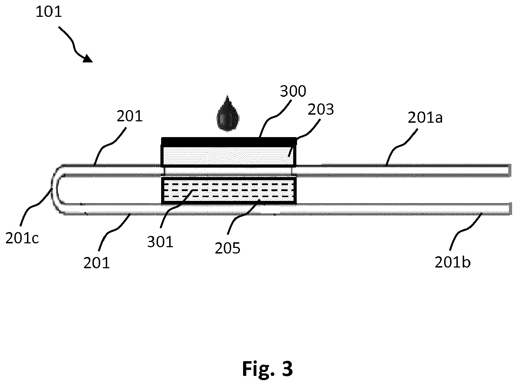

Fig. 3 shows the strip (101) of the invention in a folded position. The strip (101) is shown having a base substrate (201). The base substrate is shown folded at position (201 c) that suitably positions the first portion (201a) over the second portion (201b) of the strip (101). The suitable positioning of the first position (201a) over the second portion (201b) render the positioned set up to render a filter and a retainer. Here, the first membrane (203) of the first portion receives the blood sample administered to its surface. The first membrane (203) acts as a filter to allow only the plasma of the blood to pass through it and retain the RBCs (300) at the surface of the first membrane (203), thus separating the plasma and the RBCs of the blood sample.

The plasma passing through the first membrane (203) of the first portion (201a) of the strip (101) comes in contact with the second membrane (205) of the second portion (201b) of the strip (101). The second membrane (205) absorbs and retains the plasma (301) that is filtered and passed through the first membrane (203).

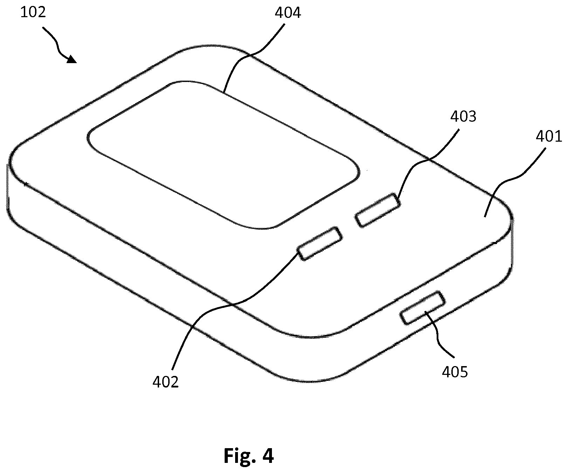

Fig. 4 shows a reader (102) for measuring bilirubin and haemoglobin, in accordance with the invention. The reader has housing (401) to accommodate various components of the reader (102). The reader (102) has selection elements (402, 403) disposed in the housing (401) and accessible to the user, which been provided to select the mode for corresponding measurement pertaining to measurement of haemoglobin or bilirubin. The selection element may vary to suit the purpose of such selection, which can also be realized with a single selection elements or with a plurality of selection elements. Here, the selection element (402) refers to Haemoglobin mode that corresponds to selecting the mode for Haemoglobin (Hb) measurement. Similarly, the selection element (403) refers to Bilirubin mode that corresponds to selecting the mode for Total Serum Bilirubin (TsB) measurement. The reader (102) has a display element (404) disposed in the housing (401) to show or display the values of the measurements or for such other purpose that suits its capability. The display (404) may be a display that includes but not limited to LCD etc. The reader (102) has a provision (405) for charging and / or powering the power management component (109) of the reader (102).

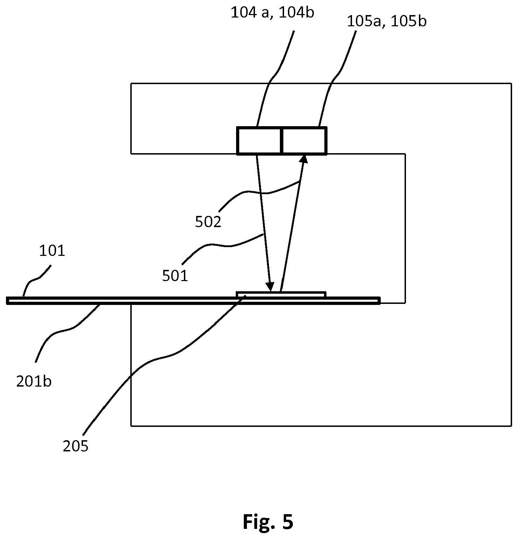

Fig. 5 depicts the illumination of the strip (101) for the measurement of bilirubin. The strip (101), especially the second membrane (205) of the second portion (201b) of the strip (101) is exposed for illumination by the light sources (104a, 104b). For the measurement of bilirubin, the blue and green lights (501) are respectively used from the light sources (104a, 104b) for illumination emitted by the light sources (104a, 104b). Blue light having a wavelength of 455 nm and green light having a wavelength of 575 nm, or of other suitable wavelengths may be used for the purpose. The selection of measurement of bilirubin can be effected by correspondingly selecting the selection element (403) of the reader (102) to effect measurement of bilirubin. The light illuminated by the light sources (104a, 104b) is reflected from the surface of the second membrane (205). The reflected lights (502) emanating from light sources (104a, 104b) strikes the light to frequency converter (105a, 105b) respectively, where its reflectance is measured. The reflectance of the reflected light (502) provides a measure of the bilirubin level. The processing unit estimates the bilirubin based on the measure of bilirubin pertaining to the reflectance.

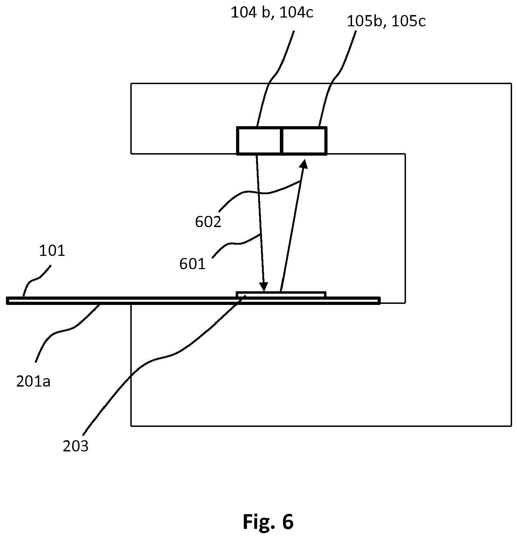

Fig. 6 depicts the illumination of the strip (101) for the measurement of haemoglobin. The strip (101), especially the first membrane (203) of the first portion (201a) of the strip (101) is exposed for illumination by the light sources (104b, 104c). For the measurement of haemoglobin, the red and green lights (601) are respectively used from the light sources (104b, 104c) for illumination emitted by the light sources (104b, 104c). Red light having a wavelength of 650 nm and green light having a wavelength of 575 nm, or of other suitable wavelengths may be used for the purpose. The selection of measurement of Haemoglobin can be effected by correspondingly selecting the selection elements (402) of the reader (102) to effect measurement of haemoglobin. The light illuminated by the light sources (104b, 104c) is reflected from the surface of the membrane (203). The reflected lights (602) emanating from light sources (104b, 104c) strikes the light to frequency converter (105b, 105c) respectively, where its reflectance is measured. The reflectance of the reflected lights (602) provides a measure of the haemoglobin level. The processing unit estimates the haemoglobin based on the measure of haemoglobin pertaining to the reflectance.

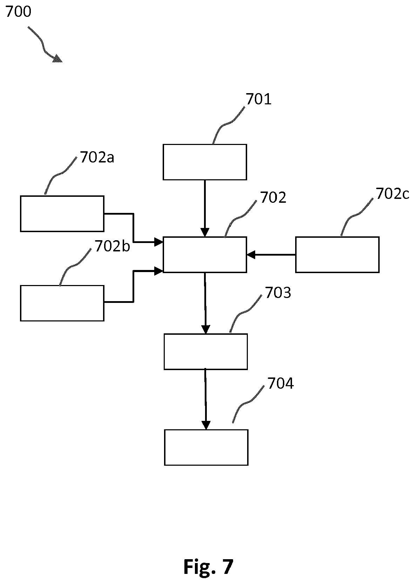

Fig. 7 represents a method (700) for measuring bilirubin and haemoglobin, in accordance with the invention. The method is described herein by way of a non-exhaustive exemplary embodiment.

The method of the invention essentially comprises the steps of separating plasma and the RBCs of the blood sample, and the step of measuring the bilirubin and / or haemoglobin.

In Fig. 7, the method (700) shows the step of administering the blood sample (701). In one embodiment, approximately 30 μl is administered. More or less could be administered, and as required. This is followed by the step of separating the plasma and the RBCs (702) of the blood sample. The step of separating the plasma and the RBCs include the step of filtering the plasma (702a) by the membrane (203) of the first portion (201a) of the strip (101), and allowing it to pass through the membrane (203). The RBCs (300) are retained (702b) on the surface of the membrane (203). The RBCs (300) may solidify to a solid state and yet suitable for measurement of the haemoglobin. The step of absorbing and retaining (702c) the plasma (301) is effected by the membrane (205) of the second portion (201b) of the strip (101). The step of illuminating the region of interest (703) purporting to the membrane (203, 205) of the first and second portions (201 a, 201b) respectively of the strip (101) is performed by the suitable light sources (104a, 104b).

Accordingly, the light sources (104a, 104b) respectively emit red and green light of suitable wavelength to illuminate the surface of the membrane (203) for the measurement of haemoglobin. Similarly, the light sources (104a, 104b) respectively emit blue and green light of suitable wavelength to illuminate the surface of the membrane (205) for the measurement of bilirubin.

The step of measuring the bilirubin and / or haemoglobin (704) includes measuring the reflectance of the light reflected from the surface of the membranes (205 or 203) by the light to frequency converter (105) of the reader (102), and estimating the haemoglobin and / or bilirubin level. The measurement of bilirubin or haemoglobin by the reader may be effected by exposing the first portion (20 1 a) or the second portion (201b) of the strip (101), especially its membrane (203 or 205) surface, which is the region of interest. This may be made possible by inserting the first portion (20 1 a) or second portion (201b) of the strip (101) into the reader exposing the same to the illumination from the light sources (104a, 104b). Other possible approaches for effecting such exposure of the first or second portion (201a, 201b) of the strip (101) to the light emanating from the light sources (104a, 104b) may also be used. Visual indication may be provided to guide and use the right kind of the strip portion in respect of the selection made for the measurement. In other words the selection of measurement whether for haemoglobin or bilirubin, through the selection elements (402, 403) may be made to correlate with the indication on the corresponding strip portion viz. first portion (20 1 a) or the second portion (201b) of the strip (101). Such indication includes but not limited to shape of the membrane i.e., first portion (201a) of the strip (101) with first membrane (203) for haemoglobin being circular in shape, and second portion (201b) of the strip (101) with second membrane (205) for bilirubin being square in shape, etc.

Measuring the bilirubin and /or haemoglobin includes measuring the reflectance of the reflected light (502 or 602) by the light to frequency converter (105), and estimating the bilirubin or haemoglobin level accordingly by the processing unit (106).

The bilirubin level is estimated as follows:

$$\text{Bilirubin level} = (-2.85*10^{(-14)}) *X^3 + 3.13*X^2 -0.011*X + 1162.76 \dots\dots$$

equation (1)

Where, X is the intensity of the reflected Blue light at 20 mA.

The haemoglobin level is estimated as follows:

$$\text{Haemoglobin Value} = 8.09*(10^{(-11)}) *X^2 -0.000111*X + 40.3$$

equation (2)

Where, X is the intensity of the reflected Green light at 20 mA.

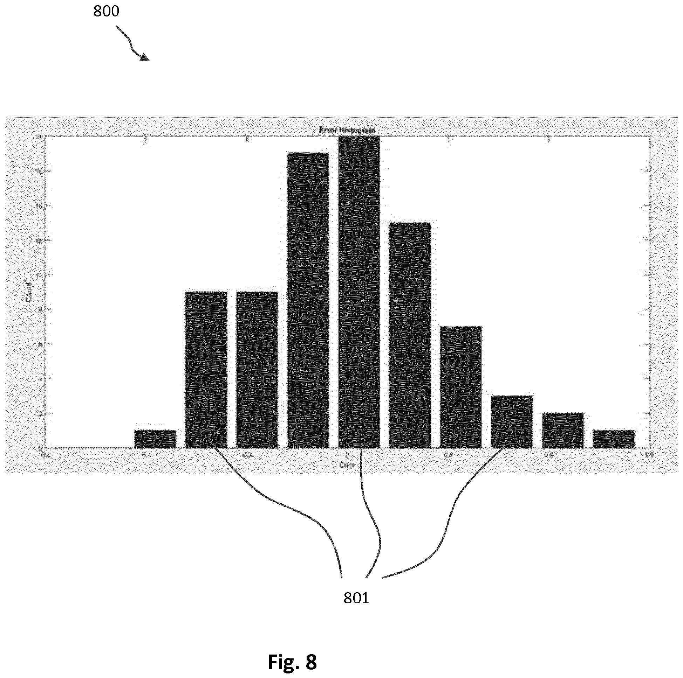

In Fig. 8, the error histogram (800) for bilirubin measurement is shown. The error is represented against the count of number of samples and shown as plots (801). The error histogram (800) is curve fitted, and gives an error of -0.4 on the lower side and 0.6 on the higher side with a standard deviation error of 0.1913.

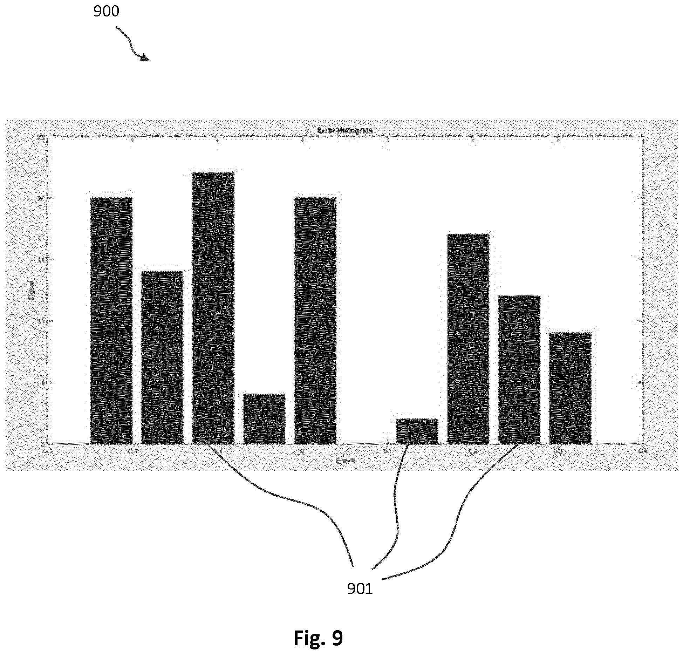

In Fig. 9, the error histogram (900) for haemoglobin measurement is shown. The error is represented against the count of number of samples and shown as plots (901). The error histogram (900) is curve fitted, and gives an error of -0.3 on the lower side and 0.3 on the higher side with a standard deviation error of 0.1839.

These errors encountered herein are significantly less, and hence the proposal of the invention is more reliable and more accurate in comparison with the other approaches for the measurement of haemoglobin and bilirubin in the context of point of care device.

The present invention also includes strip for separating plasma and RBCs in a blood sample, said strip comprising:

a base substrate foldable to form a first portion and a second portion and capable of positioning the first portion over the second portion thereof, and the first portion has a first membrane and the second portion has a second membrane, attached thereto.

The first portion of the strip can have a first membrane for filtering the plasma in the blood and for allowing the plasma to flow through it. The second portion of the strip can have a second membrane to absorb and retain the plasma.

The first membrane of the first portion of the strip may be provided for retaining the RBCs in the blood on its surface, and allowing only the plasma to pass through it. The first membrane and the second membrane may be a glass microfiber membrane. In said strip the first membrane and the second membrane may be attached to the base substrate by a suitable adhesive. The method as claimed in claim 17, wherein measuring bilirubin or haemoglobin includes illuminating the first portion or the second portion of the strip by a plurality of light sources.

Further, in the method of the present invention illuminating the first portion of the strip may include illuminating with green and red light for measuring haemoglobin; and/or illuminating the second portion of the strip may include illuminating with blue and green light for measuring bilirubin.

In addition the measuring step of the method can include measuring the reflectance of the light reflected from the surface of the first portion or second portion of the strip containing RBCs or plasma respectively, by the processing unit of the reader; and/ or include estimating the bilirubin and / or haemoglobin by the processing unit.

Only certain features of the invention have been specifically illustrated and described herein, and many modifications and changes will occur to those skilled in the art. The invention is not restricted by the preferred embodiment described herein in the description. It is to be noted that the invention is explained by way of exemplary embodiment and is neither exhaustive nor limiting. Certain aspects of the invention that not been elaborated herein in the description are well understood by one skilled in the art. Also, the terms relating to singular form used herein in the description also include its plurality and vice versa, wherever applicable. Any relevant modification or variation, which is not described specifically in the specification are in fact to be construed of being well within the scope of the invention. The appended claims are intended to cover all such modifications and changes which fall within the spirit of the invention.

Thus, it will be appreciated by those skilled in the art that the present invention can be embodied in other specific forms without departing from the spirit or essential characteristics thereof. The presently disclosed embodiments are therefore considered in all respects to be illustrative and not restricted. The scope of the invention is indicated by the appended claims rather than the foregoing description and all changes that come within the meaning and range and equivalence thereof are intended to be embraced therein.

## Claims

1. A system for measuring bilirubin and haemoglobin of a blood sample, comprising:

   a strip for separating plasma and Red Blood Cells (RBCs) in the blood sample, the strip comprising:

   a first portion having a first membrane disposed thereon;
   a second portion, disposed from the first portion by a foldable portion and having a second membrane disposed thereon;
   wherein the first portion is disposed over the second portion in a folded position.

2. The system as claimed in claim 1, further comprising a reader for reading at least one of a bilirubin level or haemoglobin level from the strip

3. The system as claimed in claim 1 or 2, wherein the first membrane includes dimensions that allow plasma from the blood sample to flow through it.

4. The system as claimed in claim 1 or 2, wherein the second membrane has dimensions that absorb and retain the plasma.

5. The system as claimed in claim 1 or 2, wherein the first membrane of the first portion of the strip is provided for retaining the RBCs in the blood on its surface, and allowing only the plasma to pass through it.

6. The system as claimed in claims 1 to 5, wherein the first membrane and the second membrane is a glass microfiber membrane.

7. The system as claimed in claim 2, wherein the reader has a plurality of light source provided for illuminating the first portion or the second portion of the strip, for measuring the haemoglobin or bilirubin respectively, based on reflectance photo spectrometry.

8. The system as claimed in claim 6, wherein the light source provides illumination with blue and green light for measuring bilirubin.

9. The system as claimed in claim 6, wherein the light source provides illumination with green and red light for measuring haemoglobin.

10. The system as claimed in claims 2 and 7 to 9, wherein the reader has a processing unit configured to measure the reflectance of the light reflected from the surface of the first portion or second portion of the strip containing RBCs or plasma respectively.

11. The system as claimed in claim 10, wherein the processing unit is provided for estimating the bilirubin and / or haemoglobin.

12. A method for measuring bilirubin and haemoglobin of a blood sample, comprising:

separating plasma and Red Blood Cells (RBCs) in the blood sample by a strip that is foldable to form a first portion and a second portion and capable of positioning the first portion over the second portion thereof, and the first portion has a first membrane and the second portion has a second membrane, attached thereto; and measuring bilirubin or haemoglobin level or both, by a reader.

13. The method as claimed in claim 12, wherein separating plasma and RBCs include filtering the plasma in the blood and allowing the plasma to flow through the first membrane of the first portion of the strip.

14. The method as claimed in claim 12, wherein separating plasma and RBCs include absorbing and retaining the plasma by the second membrane of the second portion of the strip.

15. The method as claimed in claim 12, wherein separating plasma and RBCs include retaining the RBCs in the blood on the surface of the first membrane of the first portion of the strip and allowing only the plasma to pass through it.

**Fig. 1**

**Fig. 2**

**Fig. 3**

102      404      403      401

402      405

**Fig. 4**

104 a, 104b      105a, 105b

502

501

101

201b

205

**Fig. 5**

**Fig. 6**

700

702a

701

702

702c

702b

703

704

**Fig. 7**

EP 3 470 839 A1

800

Fig. 8

801

16

900

Fig. 9

Europäisches
Patentamt

European
Patent Office

Office européen
des brevets

# EUROPEAN SEARCH REPORT

**Application Number**

EP 17 19 6065

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/025726 A1 (VIVEBIO LLC [US]) 18 February 2016 (2016-02-18) | 1-11 | INV. G01N33/49 |
| Y | * paragraph [0002] * * paragraph [0010] - paragraph [0028] * * figures * * paragraph [0079] * | 12-15 | G01N33/487 G01N33/72 |
| Y | WO 2012/038930 A1 (FOND ITALIANA FEGATO ONLUS [IT]; WENNBERG RICHARD PARTRIDGE [US]; MICR) 29 March 2012 (2012-03-29) | 12-15 | |
| A | * page 1, line 13 - page 5, line 25 * * page 6, line 19 - page 7, line 26; figures * | 1-11 | |
| A | EP 0 336 483 A1 (X FLOW BV [NL]; PRIMECARE BV [NL]) 11 October 1989 (1989-10-11) * page 7, line 42 - page 8, line 29; figures * | 1-15 | |

**TECHNICAL FIELDS SEARCHED (IPC)**

G01N

The present search report has been drawn up for all claims

| Place of search | Date of completion of the search | Examiner |
|---|---|---|
| The Hague | 26 February 2018 | Savage, John |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.......................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 19 6065

This annex lists the patent family members relating to the patent documents cited in the above-mentioned European search report.
The members are as contained in the European Patent Office EDP file on
The European Patent Office is in no way liable for these particulars which are merely given for the purpose of information.

26-02-2018

| Patent document cited in search report | | Publication date | Patent family member(s) | | Publication date |
|---|---|---|---|---|---|
| WO 2016025726 | A1 | 18-02-2016 | NONE | | |
| WO 2012038930 | A1 | 29-03-2012 | NONE | | |
| EP 0336483 | A1 | 11-10-1989 | AT | 108021 T | 15-07-1994 |
| | | | CA | 1322335 C | 21-09-1993 |
| | | | DE | 68916458 D1 | 04-08-1994 |
| | | | DE | 68916458 T2 | 01-12-1994 |
| | | | EP | 0336483 A1 | 11-10-1989 |
| | | | ES | 2058470 T3 | 01-11-1994 |
| | | | JP | 2729503 B2 | 18-03-1998 |
| | | | JP | H01302161 A | 06-12-1989 |
| | | | NL | 8800796 A | 16-10-1989 |
| | | | US | 5240862 A | 31-08-1993 |

EPO FORM P0459

For more details about this annex : see Official Journal of the European Patent Office, No. 12/82